# Europäisches Patentamt

**European Patent Office**

**Office européen des brevets**

(11) Numéro de publication: **0 170 540**
**B1**

(12) **FASCICULE DE BREVET EUROPEEN**

(45) Date de publication du fascicule du brevet: 26.09.90

(51) Int. Cl.⁵: **A 61 K 9/52,** A 61 K 9/50, B 01 J 13/02

(21) Numéro de dépôt: 85401087.3

(22) Date de dépôt: 03.06.85

(54) Procédé de préparation de microcapsules biodégrables à base de sérumalbumine.

(30) Priorité: 05.06.84 FR 8408761

(43) Date de publication de la demande:
05.02.86 Bulletin 86/06

(45) Mention de la délivrance du brevet:
26.09.90 Bulletin 90/39

(84) Etats contractants désignés:
AT BE CH DE FR GB IT LI LU NL SE

(56) Documents cités:
EP-A-0 054 396
FR-A-2 326 934
LU-A- 76 237

(73) Titulaire: UNIVERSITE PARIS-SUD (PARIS XI)
15, rue Georges Clémenceau
F-91405 Orsay Cédex (FR)

(72) Inventeur: Fickat, René
4 Groupe Général Eisenhower
F-51100 Reims (FR)
Inventeur: Benita, Simon
37, rue de la République Apt. 207
F-92800 Puteaux (FR)
Inventeur: Puisieux, Francis
66 rue de Strasbourg
F-94700 Maisons-Alfort (FR)

(74) Mandataire: Ahner, Francis et al
CABINET REGIMBEAU, 26, avenue Kléber
F-75116 Paris (FR)

EP 0 170 540 B1

Courier Press, Leamington Spa, England.

# EP 0 170 540 B1

**Description**

La présente invention concerne un procédé de préparation de microcapsules à base de sévumalbumine renfermant une substance pharmaceutiquement active.

Il convient tout d'abord de rappeler que les microcapsules sont des organites artificiels qui sont utilisés pour la mise en forme galénique de divers médicaments. L'inclusion d'un principe actif dans une sphérule microscopique permet notamment d'assurer sa protection transitoire vis-à-vis de divers agents dénaturants, tels que les enzymes digestives.

Dans d'autres cas, la paroi des microcapsules permet de moduler la diffusion du principe actif, ce qui est mis à profit dans l'élaboration de formes galéniques à action retardée. Il est ainsi nécessaire que la paroi de la microcapsule offre toutes les garanties d'innocuité liées à l'administration en thérapeutique humaine.

De telles exigences sont d'autant plus sévères que les microcapsules sont destinées à des thérapies in situ, telle que la chimioembolisation.

On rappellera brièvement que la chimioembolisation est une technique que l'on envisage d'utiliser dans le traitement de certaines tumeurs. Selon cette technique, l'artère nourricière de la tumeur est oblitérée à l'aide d'un embole mis en place par cathétérisme. En chimioembolisation, l'embole contient une substance antitumorale qui diffuse dans les tissus tumoraux.

Dans la technique antérieure on a déjà proposé des microcapsules dont la paroi externe est obtenue par réticulation interfaciale de diverses protéines, notamment choisies parmi les protéines d'origine naturelle afin d'offrir une bonne garantie d'innocuité.

Bien que diverses tentatives aient été faites dans la technique antérieure, on n'est jamais parvenu à préparer des sphérules totalement biodégradables in vivo.

Les résultats les plus performants semblent être obtenus avec des microcapsules de sérumalbumine, qui, lorsqu'elles sont injectées dans l'artère rénale du rat, présentent une bonne tolérance, mais une durée de dégradation in vivo bien trop longue.

Une telle durée de dégradation qui dépasse toujours 15 jours, provoque des effets secondaires systémiques indésirables, notamment de type inflammatoire, qui interdisent l'utilisation de telles formes de dosage, en particulier dans le domaine de la chimioembolisation. Une dégradation trop lente des microcapsules, supérieure à environ 8 jours, conduit à la formation de lésions ischémiques irréversibles de l'organe situé en aval de l'artère nourricière oblitérée au moyen des microcapsules mises en place par cathétérisme.

De surcroît, après un séjour des microcapsules dans l'artère nourricière supérieur à environ 8 jours, on observe la formation d'un granulome inflammatoire donnant naissance à une sorte d'agrégat sclérotique qui réduit considérablement la libération du principe actif encapsulé.

En ce qui concerne la préparation de telles microcapsules la technique antérieure est plus particulièrement illustrée par le brevet FR 2 326 934. Ce brevet préconise dans une première étape la dissolution ou la dispersion du principe actif directement dans une solution aqueuse de la sérumalbumine. Les microcapsules ainsi obtenues sont ensuite, dans une seconde étape, mises en présence simultanément d'un alcool et d'un agent de réticulation.

En fait, un tel procédé implique que l'agent de réticulation choisi soit compatible avec le principe actif préalablement incorporé par dissolution dans les microcapsules et en conséquence a une application limitée.

La présente invention a précisément pour but d'éviter ces inconvénients et de proposer un procédé permettant d'incorporer une grande variété de principes actifs par diffusion à l'intérieur des parois de ces microcapsules, et également d'abaisser le temps de dégradation in vivo des microcapsules à moins de 8 jours.

Cet objectif a pu être atteint, conformément à la présente invention, par la mise au point d'un procédé de préparation de microcapsules biodégradables renfermant une substance pharmaceutiquement active, caractérisé en ce que la paroi des microcapsules est constituée par de la sérumalbumine qui est en partie réticulée par polymérisation interfaciale à l'aide d'un réactif bifonctionnel acylant, et pour l'autre partie non réticulée, dénaturée à l'aide d'un alcool.

Il convient en outre de préciser que selon ce procédé de préparation, il a été possible d'obtenir outre une parfaite biodégradabilité des microcapsules, une excellent capacité d'absorbtion des principes actifs, propriété qui n'a jamais pu être obtenue dans la technique antérieure.

Conformément à la présente invention, le procédé pour préparer les microcapsules à base de sérumalbumine se caractérise par la mise en oeuvre des opérations successives suivantes:

a) la sérumalbumine est émulsifiée par dispersion au sein d'un système solvant organique, par addition d'un agent émulsifiant pharmaceutiquement acceptable, et par agitation;

b) à l'émulsion ainsi obtenue, maintenue sous agitation, on ajoute un agent de réticulation en solution dans ledit système solvant organique et l'on maintient l'agitation jusqu'à obtention du degré de réticulation interfaciale désirée;

c) on isole les microcapsules après dilution du mélange réactionnel, suivie d'une succession d'étapes de lavage avec des solvants appropriés et d'étapes de décantation, puis on soumet les microcapsules à plusieurs lavages à l'alcool et à une immersion dans l'alcool, de préférence absolu, pendant au moins 12 heures afin de dénaturer la sérumalbumine non réticulée, et

2

d) après séchage des microcapsules, on y incorpore la substance pharmaceutiquement active par immersion contrôlée des microcapsules dans une solution titrée de ladite substance.

D'autres caractéristiques et avantages de la présente invention apparaîtront à la lecture de la description faite ci-après, notamment en référence au dessin annexé sur lequel les figures 1 à 3 représentent des clichés de microcapsules selon l'invention vu au microscope électronique à balayage (sur la figure 1: 2,2 cm représentent 200 µm; sur la figure 2: 1,9 cm représente 200 µm; sur la figure 3: 2,4 cm représentent 200 µm).

Le procédé selon la présente invention permettant la préparation de microcapsules se subdivise donc principalement en 4 étapes successives, à savoir:

a) émulsification de la sérumalbumine;
b) réticulation interfaciale;
c) isolement des microcapsules, et
d) incorporation du principe actif.

Au cours de la première étape d'émulsification de la sérumalbumine, on disperse cette dernière au sein d'un système solvant organique, grâce à l'adjonction d'un agent émulsifiant pharmaceutiquement acceptable et en réalisant une agitation. La sérumalbumine utilisée est par exemple obtenue par lyophilisation d'une solution d'albumine sérique humaine à 20%, se présentant sous la forme d'une poudre jaune pâle entièrement soluble dans l'eau.

Le système solvant organique utilisé au cours de cette première étape d'émulsification est avantageusement choisi de telle sorte qu'il ne soit pas missible à l'eau, et que l'agent de réticulation utilisé au cours de la seconde étape puisse s'y dissoudre. On emploie avec profit par exemple un mélange binaire constitué par un hydrocarbure chloré à 1 ou 2 atomes de carbone, tel que le chloroforme, le chlorure de méthylène ou le trichloroéthylène, et par un hydrocarbure non chloré comprenant de 5 à 7 atomes de carbone, tel que l'hexane ou le cyclohexane.

L'agent émulsifiant utilisé au cours de cette première étape est un agent tensio-actif tel qu'un ester de sorbitane et d'acide gras ou tout autre tensio-actif permettant de préparer une émulsion du type eau dans l'huile. Le trioléate de sorbitane (Span 85®) peut ainsi être utilisé. Dans la pratique on préfère toutefois faire appel à la phosphatidylcholine (Epikuron 180®).

Les volumes et quantités relatifs de sérumalbumine, de solvants organiques et d'agent émulsifiant permettant d'obtenir l'émulsion destinée à être réticulée ultérieurement, peuvent être adaptés avec une assez grande latitude, et s'éloigner notablement des proportions indiquées ultérieurement à simple titre d'exemple.

Une telle émulsion peut aisément être obtenue par simple agitation, par exemple au moyen d'une ancre de verre entraînée mécaniquement à l'intérieur d'un tube de centrifugation. Une vitesse de rotation de l'ordre de 500 à 1000 tours/minute, poursuivie pendant environ 1 à 3 minutes suffit alors pour mener à bien l'émulsification.

Au cours de la deuxième étape, l'émulsion ainsi obtenue et maintenue sous agitation, est soumise à une réticulation interfaciale. Au cours de cette étape, la paroi des microcapsules est formée par réticulation, en particulier au moyen d'un réactif bifonctionnel acylant, tel qu'un halogénure de diacide carboxylique, un diisocyanate, où les deux groupes fonctionnels sont tous deux portés par un cycle aromatique, ou bien séparés par une chaîne aliphatique de 2 à 10 atomes de carbone, ou portés, l'un par un cycle aromatique, l'autre par une chaîne aliphatique juxtanucléaire. Il peut par exemple s'agir du dichlorure de succinyle, du dichlorure de sébacoyle, du dichlorure de téréphtaloyle, du diisocyanate de toluène, du diisocyanate d'hexaméthylène.

Cet agent de réticulation est ajouté à l'émulsion, sous la forme d'une solution dans le même système solvant organique que celui utilisé au cours de la première étape d'émulsification. La réaction de polymérisation interfaciale est conduite à une température qui peut être comprise entre le point de congélation et le point d'ébullition des liquides utilisés, le plus souvent à température ordinaire.

Tel qu'énoncé précédemment, il est apparu que le temps de réticulation interfacial devait être compris entre environ 30 minutes et environ 90 minutes, des résultats parfaitement satisfaisants ayant été obtenus dans la pratique avec des temps de réticulation interfaciale de l'ordre de 60 minutes.

En outre, conformément à une caractéristique additionnelle de la présente invention, le rapport pondéral de la sérumalbumine à l'agent de réticulation est choisi tel que:

$$\frac{1}{1,250} < \frac{\text{sérumalbumine}}{\text{agent de réticulation}} < \frac{1}{0,2}$$

Dans tout cette gamme de proportions relatives entre la sérumalbumine et l'agent de réticulation on obtient pour des durées de réticulation interfaciale de l'ordre de 60 minutes, des microcapsules entièrement biodégradables in vivo. Il est cependant parfaitement clair que les propriétés de cette paroi, ainsi d'ailleurs que son aspect, se trouvent cependant modifiées en fonction de ce rapport.

Au cours de la troisième étape du procédé de l'invention on isole les microcapsules du mélange réactionnel, après avoir dilué ce dernier en vue de stopper la réaction de réticulation. Alors que dans la technique antérieure l'opération d'isolement des microcapsules constituait une étape ne présentant aucune difficulté réelle, les microcapsules, objets de la présente invention, ne peuvent être éliminées du milieu réactionnel que de façon beaucoup plus délicate.

Dans la technique antérieure, après avoir arrêté l'agitation, les microcapsules étaient laissées à sédimenter, puis lavées par une solution de Tween 20® dans de la glycérine à 25% et plusieurs mélanges successifs d'eau et d'alcool. Après chaque lavage, les microcapsules étaient alors isolées par filtration ou centrifugation, puis séchées.

Une telle méthode de séparation convenait aux microcapsules dont la paroi était constituée par une protéine fortement réticulée. Cette paroi était en effet assez résistante pour supporter les lavages successifs et l'opération de centrifugation. En revanche, les microcapsules partiellement réticulées, obtenues selon le procédé de la présente invention, possèdent une paroi de faible résistance, qui éclate à la suite des différents lavages aqueux et ne supporte en aucun cas la centrifugation.

Ainsi il a fallu faire appel à une méthode de séparation évitant pratiquement le contact avec l'eau, et les chocs de gravitation.

Conformément au procédé de la présente invention, les microcapsules sont isolées du milieu réactionnel par mise en oeuvre des opérations successives suivantes:
— on sépare le surnageant;
— on effectue au moins un lavage des microcapsules à l'aide dudit système solvant organique;
— on ajoute aux microcapsules, dans l'ordre 1/3 de glycérine contenant environ 20% de polysorbate, puis 2/3 d'eau; et
— on sépare les microcapsules par simple sédimentation avant de procéder au lavage à l'alcool et à l'immersion dans l'alcool desdites microcapsules.

Il est important d'ajouter aux microcapsules d'abord la glycérine contenant le polysorbate puis l'eau. En effet, la glycérine provoque autour des microcapsules une sorte de film protecteur. Au cours de toutes ces opérations de séparation, les microcapsules sont modérément agitées puis séparées par sédimentation.

En fin de préparation, il est indispensable de procéder au lavage des microcapsules par de l'alcool contenant éventuellement une faible proportion d'eau. Dans la pratique, une succession de 5 lavages avec des mélanges hydroalcooliques contenant respectivement 60%, 70%, 80%, 90% et 95% d'alcool absolu, conduisent à une parfaite élimination de toutes traces d'agents de réticulation résiduels.

A ce stade, les microcapsules faiblement réticulées se dégradent très rapidement in vivo, mais sont encore très avides d'eau au contact de laquelle elles éclatent spontanément.

Conformément au procédé de la présente invention, les microcapsules ainsi lavées sont immergées pendant au moins 12 heures dans de l'alcool, de préférence absolu, de manière à compléter la dénaturation de l'albumine qui n'a pas été réticulée.

Enfin, on sépare les microcapsules par évaporation de l'alcool, par exemple dans un évaporateur rotatif, à une température de l'ordre de 35°C, puis on laisse les microcapsules, par exemple à 35°C pendant 24 heures dans une étuve afin de compléter le séchage. Les microcapsules ainsi obtenues, même très faiblement réticulées, ont alors une paroi résistante qui permet leur manipulation aisée, les microcapsules ainsi obtenues étant en outre devenues parfaitement stables dans l'eau.

En se reportant au dessin annexé, on constate que les microcapsules obtenues selon le procédé de la présente invention, peuvent présenter des morphologies différentes traduisant des degrés de réticulation différents.

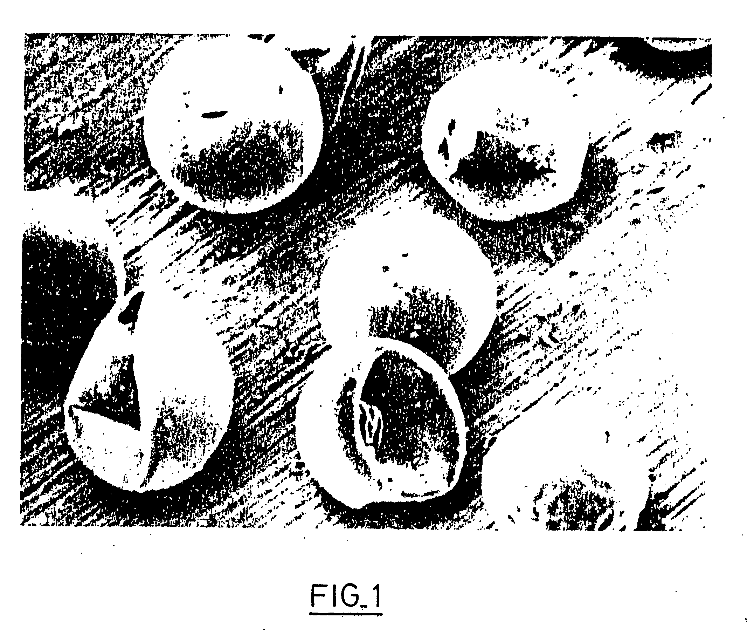

Ainsi, les microcapsules de la figure 1 résultent d'une polymérisation interfaciale conduite avec un rapport sérumalbumine sur agent de réticulation égal à 1/1,250. Leur surface de paroi est lisse. Ces microcapsules sont parfaitement biodégradables, cependant leur durée de dégradation se situe aux alentours de 15 jours, ce qui interdirait par conséquent leur utilisation en chimioembolisation.

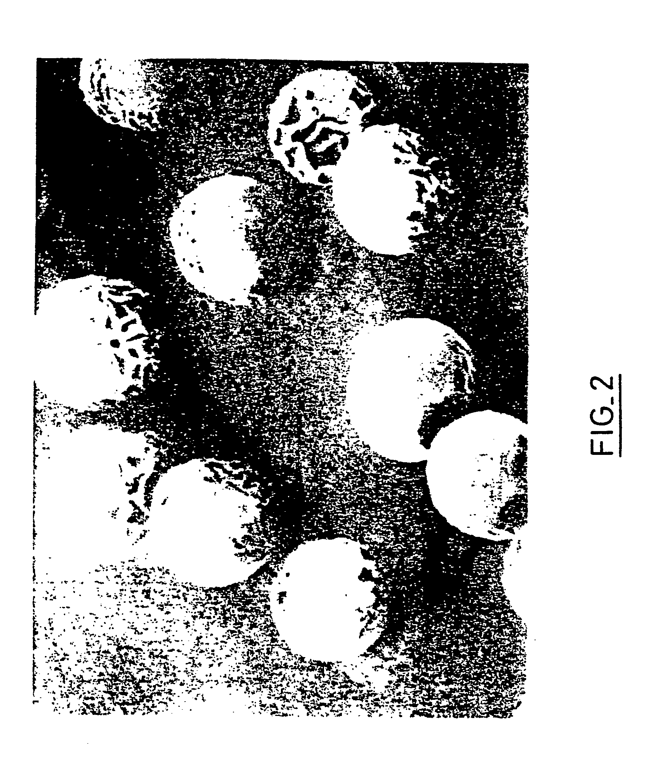

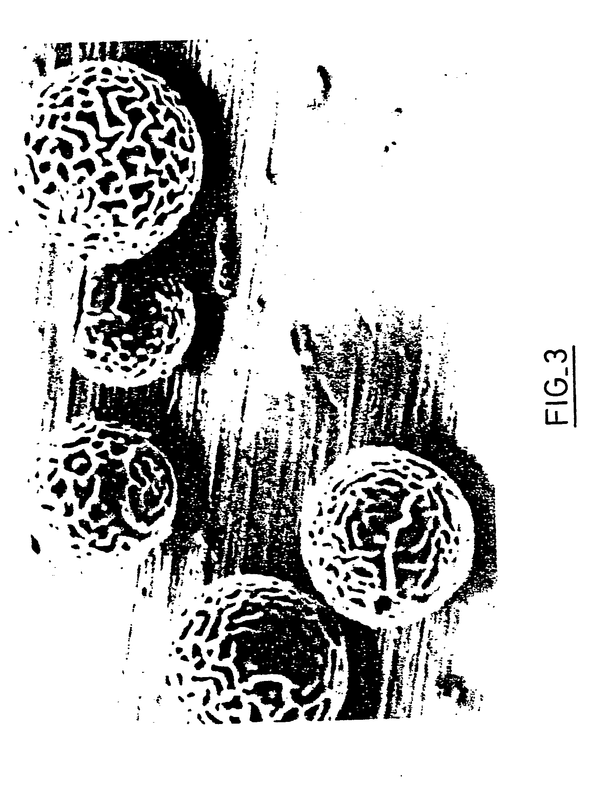

Les microcapsules des figures 2 et 3 correspondent respectivement à des rapports sérumalbumine/ agent de réticulation égal à:

$$\frac{1}{0,250} \text{ (figure 2)} \quad \text{et} \quad \frac{1}{0,200} \text{ (figure 3)}$$

Ces microcapsules présentent une paroi rugueuse d'aspect frippé qui résulte du séjour des microcapsules dans l'alcool qui dénature les régions non réticulées. On précisera à ce propos que les microcapsules faiblement réticulées présentent un aspect initial jaune avant leur immersion dans l'alcool, alors qu'après immersion elles deviennent blanches.

Avantageusement, les microcapsules ainsi obtenues sont soumises à une opération de stérilisation, par exemple une radiostérilisation par application d'un rayonnement d'environ 2,5 M rads.

L'analyse des microcapsules ainsi obtenues démontre que la totalité de l'agent de réticulation a été éliminé, alors que dans le cas des microcapsules de la technique antérieure, une faible quantité d'agents de réticulation se trouve toujours retenue sur les parois des microcapsules.

# EP 0 170 540 B1

Dans le cadre de la présente invention, l'élimination totale de l'agent de réticulation est en partie au moins due à l'immersion prolongée des microcapsules dans l'alcool.

## EXEMPLE DE PREPARATION DE MICROCAPSULES

1000 mg de sérumalbumine humaine lyophilisée sont dissous dans 3 ml d'un tampon $Na_2CO_3$ (pH = 9,8 ajusté avec de l'acide chlorhydrique). Cette solution est émulsifiée dans 15 ml d'un système solvant organique constitué par un mélange binaire chloroforme:cyclohexane (1:4) contenant 0,5% de phosphatidylcholine. Une agitation contrôlée correspondant à environ 500 tours/minutes est maintenue pendant environ 60 secondes.

A cette émulsion ainsi obtenue on ajoute 15 ml d'une solution de chlorure de téréphtaloyle à 1,25—2,5% dans le même système solvant chloroforme:cyclohexane (1:4).

La réticulation de la sérumalbumine dissoute dans les gouttelettes de tampon dispersé s'effectue à l'interface avec le système solvant chloroforme:cyclohexane. On poursuit la réaction de réticulation dans ces conditions pendant environ 60 minutes. Puis la suspension des microcapsules est diluée avec 30 ml du même système solvant, afin de stopper la réticulation.

Après élimination du surnageant, les microcapsules ont d'abord été rincées avec une solution de glycérine contenant 25% de polysorbate 20, puis avec de l'eau. On procède ensuite à plusieurs lavages successifs, par exemple à l'aide de 5 fois 50 ml de solutions hydroalcoolique ou éventuellement d'alcool absolu. Les microcapsules ainsi lavées sont immergées pendant au moins 12 heures dans de l'alcool de préférence absolu, par exemple de l'alcool éthylique.

Les microcapsules ainsi obtenues sont alors chargées en principe actif, par une simple opération d'immersion contrôlée des microcapsules dans une solution titrée du médicament.

La substance pharmaceutiquement active adsorbée par les parois des microcapsules est de préférence constituée par un composée hydrosoluble, ayant un poids moléculaire sensiblement compris entre 300 et 600, et portant une charge électrique.

A titre d'exemple particulier de substances pharmaceutiquement actives pouvant être adsorbées sur les parois des microcapsules selon la présente invention, on citera la doxorubicine, de préférence utilisée sous la forme de son chlorhydrate.

Un tel médicament pourra en particulier être utilisé dans le domaine de la chimioembolisation. L'invention n'est toutefois par limitée à l'utilisation de ce médicament particulier, d'autres principes actifs, notamment à activité anti-néoplastique, pouvant également être incorporés dans les microcapsules.

Dans la pratique, il s'est avéré que la libération du principe actif à partir de ces microcapsules chargées est rapide et s'effectue par diffusion. La vitesse de libération diminue uniquement pour des principes actifs dont le poids moléculaire est supérieur à 1000 Daltons. Il est intéressant de noter que le pouvoir d'adsorbtion et d'incorporation des microcapsules obtenues selon le procédé de la présente invention, augmente quand la vitesse de biodégradation augmente.

## ETUDE SUR LA BIODEGRADABILITE

L'étude préliminaire de biodégradabilité a été réalisée sur trois chiens en utilisant la méthode décrite par Madoulé Ph., Trampont Ph., Quillard J., Doyon D., Lanol-Jeantet M., Zouaï O., et Tilleul P., Expérimentation de nouveaux matériaux d'embolisation Sci, Tech., Pharm., T.II n° 10, 10, 441—445, 1982.

Les chiens ont été embolisés au niveau du rein gauche avec des microcapsules de la présenté invention, les reins droits servant de témoins. Avec des microcapsules faiblement réticulées, notamment obtenues avec un rapport sérumalbumine/agent de réticulation compris entre 1/0,7 et 1/0,2, les résultats observés 7 jours après l'embolisation indiquent qu'il y a une bonne revascularisation due à une dégradation totale des microcapsules à l'intérieur de cette période de 7 jours. Après une période plus longue d'environ 10 jours la revascularisation est parfaite et le rein redevient fonctionnel. Après sacrifice du chien et analyse histologique, aucune anomalie n'est à signaler, à l'exception d'une petite zone hémorragique située au niveau du pôle supérieur du rein. A titre de comparaison, en faisant appel à des microcapsules de sérumalbumine entièrement réticulées, ce n'est qu'après une période d'environ 15 jours que la revascularisation est amorcée, ce qui indique le début de la dégradation in vivo des microcapsules.

Après sacrifice des animaux et analyse histologique du rein, on observe une diminution du volume du pôle supérieur du rein et une augmentation du volume du pôle inférieur avec des zones hémorragiques importantes à l'intérieur du rein. Ces observations comparatives révèlent l'avantage déterminant des microcapsules, objets de la présente invention, par rapport aux microcapsules entièrement réticulées de la technique antérieure.

## ETUDE DE LA STRUCTURE INTERNE DES MICROCAPSULES

Cette étude a été effectuée à l'aide de la microscopie électronique à balayage.

Il s'agit d'un système réservoir constitué d'une paroi de sérum-albumine partiellement réticulée délimitant une cavité.

5

ETUDE DE L'ADSORPTION DE MEDICAMENTS OBTENUS SELON LE PROCEDE DE PREPARATION DE L'INVENTION

1. Méthode

100 mg de microcapsules sont mis en suspension dans 125 ml d'une solution de chlorhydrate d'adriamycine. Le milieu est agité à 200 tpm. L'appareillage est gardé à l'abri de la lumière. L'adsorption de l'adriamycine sur les microcapsules est suivie par diminution de la concentration en médicament dans le surnageant (spectrophotométrie à 492 nm).

2. Résultats

A partir d'une solution de chlorhydrate d'adriamycine à 0,2 mg/ml, les microcapsules obtenues selon le procédé de la présente invention, fixent 90% à 95% du médicament en une heure.

Un degré de réticulation supérieur à 0,250 g de chlorure de téréphtaloyle pour 1 g d'albumine, ne permet pas cette fixation. De plus, pour un degré de réticulation de 0,250 g de chlorure de téréphtaloyle pour 1 g d'albumine, le temps de polycondensation interfaciale a une grande influence. Un temps de polymérisation compris entre 30 et 60 minutes permet une fixation de 90 à 95% d'adriamycine chlorhydrate en une heure. Un temps de polymérisation de 90 minutes ne permet qu'une fixation de 16% du médicament dans le même temps (1 heure).

Ces microcapsules fixent, également dans les mêmes conditions, de petits ions minéraux tels que le calcium ($Ca^{\oplus\oplus}$). Il faut, toutefois, que ces ions soient chargés positivement. Un ion thiocyanate ($-S=C=N^{\ominus}$), par exemple, ne s'adsorbe pas sur ces particules.

ETUDE DE LA LIBERATION IN VITRO DE MEDICAMENTS A PARTIR DES MICROCAPSULES DE SERUM-ALBUMINE

1. Méthode

100 mg de microcapsules chargées en adriamycine sont mis en suspension dans 250 ml de milieu de dissolution sous agitation (100 tpm), à température ambiante et à l'abri de la lumière. La présence du médicament dans le milieu de dissolution est suivie par spectrophotométrie à 492 nm.

2. Résultats

Les microcapsules ont été mises en suspension 1) dans l'eau distillée, 2) dans du sérum physiologique (NaCl 0,9%). Dans l'eau distillée, moins de 5% du médicament sont libérés dans la phase aqueuse en 24 heures. La présence d'un sel tel que le chlorure de sodium, permet la libération de l'adriamycine en une période de 10 heures. La quantité totale de médicament libérée pour 100 mg de microcapsules, dépend du volume de sérum physiologique en tant que milieu de libération (Tableau 1).

## Tableau 1

Influence du volume du milieu de dissolution sur la quantité d'adriamycine libérée des microcapsules de sérum-albumine

| Volume d'une solution de NaCl à 0,9 % (ml) | Quantité d'adriamycine libérée (%) des micro-capsules de sérum albumine |
|---|---|
| 250 | 55,0 |
| 500 | 70,5 |
| 1000 | 81,3 |

Au regard des expériences de libération in vitro, le comportement de l'adriamycine vis-à-vis des microcapsules, montre que les sites d'adsorption des microcapsules sont chargés négativement.

De plus, les études d'adsorption de l'adriamycine montrent clairement que le degré de réticulation de la paroi a une influence significative sur la pénétration du médicament dans la paroi des microcapsules. Ces observations conduisent à la conclusion que ces microcapsules se comportent comme des résines échangeuses d'ions vis-à-vis des médicaments destinés à être véhiculés.

6

**Revendications**

1. Procédé de préparation de microcapsules renfermant une substance pharmaceutiquement active, caractérisé en ce que l'on prépare les microcapsules à partir de sérumalbumine par mise en oeuvre des opérations successives suivantes:

a) la sérumalbumine est émulsifiée par dispersion au sein d'un système solvant organique, par addition d'un agent émulfisiant pharmaceutiquement acceptable, et par agitation;

b) à l'émulsion ainsi obtenue, maintenue sous agitation, on ajoute un agent de réticulation en solution dans ledit système solvant organique et l'on maintient l'agitation jusqu'à obtention du degré de réticulation interfaciale désirée;

c) on isole les microcapsules après dilution du mélange réactionnel, suivie d'une succession d'étapes de lavage avec des solvants appropriés et d'étapes de décantation, puis on soumet les microcapsules à plusieurs lavages à l'alcool et à une immersion dans l'alcool, de préférence absolu, pendant au moins 12 heures afin de dénaturer la sérumalbumine non réticulée, et

d) après séchage des microcapsules, on y incorpore la substance pharmaceutiquement active par immersion contrôlée des microcapsules dans une solution titrée de ladite substance.

2. Procédé selon la revendication 1, caractérisé en ce que l'on isole des microcapsules par mise en oeuvre des opérations successives suivantes:

on sépare le surnageant;

on effectue au moins un lavage des microcapsules à l'aide dudit système solvant organique;

on ajoute aux microcapsules, dans l'ordre, 1/3 de glycérine contenant environ 25% de polysorbate, puis 2/3 d'eau;

on sépare les microcapsules par sédimentation avant de procéder audit lavage à l'alcool et à l'immersion dans l'alcool.

3. Procédé selon l'une des revendications 1 ou 2, caractérisé en ce que l'agent émulsifiant est la phosphatidylcholine.

4. Procédé selon l'une des revendications 1 à 3, caractérisé en ce que le système solvant organique est constitué par un mélange binaire d'un hydrocarbure chloré en $C_1$ ou $C_2$ et d'un hydrocarbure non chloré en $C_5$ à $C_7$.

5. Procédé selon la revendication 4, caractérisé en ce que le système solvant organique est constitué par un mélange de chloroforme et de cyclohexane.

6. Procédé selon l'une des revendications 1 à 5, caractérisé en ce que l'agent de réticulation est un réactif bifonctionnel acylant, tel qu'un halogénure de diacide carboxylique ou un diisocyanate, dans lequel les deux groupements fonctionnels sont tous deux portés par un cycle aromatique ou bien séparés par une chaîne aliphatique contenant de 2 à 10 atomes de carbone, ou portés, l'un par un cycle aromatique et l'autre par une chaîne aliphatique juxtanucléaire.

7. Procédé selon la revendication 6, caractérisé en ce que l'agent de réticulation est choisi parmi le dichlorure de succinyle, le dichlorure de sébacoyle, le dichlorure de téréphtaloyle, le diisocyanate de toluène et le diisocyanate d'hexaméthylène.

8. Procédé selon l'une des revendications 1 à 7, caractérisé en ce que le temps de réticulation interfaciale est compris entre environ 30 minutes et environ 90 minutes.

9. Procédé selon la revendication 8, caractérisé en ce que le temps de réticulation interfaciale est d'environ 60 minutes.

10. Procédé selon l'une des revendications 1 à 9, caractérisé en ce que le rapport pondéral de la sérumalbumine à l'agent de réticulation est choisi tel que:

$$\frac{1}{1,250} < \frac{\text{sérumalbumine}}{\text{agent de réticulation}} < \frac{1}{0,2}$$

11. Procédé selon la revendication 10, caractérisé en ce que le rapport pondéral de la sérumalbumine à l'agent de réticulation est choisi tel que:

$$\frac{1}{0,7} < \frac{\text{sérumalbumine}}{\text{agent de réticulation}} < \frac{1}{0,2}$$

12. Procédé selon l'une des revendications 1 à 11, caractérisé en ce que l'alcool utilisé pour les opérations de lavage et d'immersion des microcapsules est constitué par de l'alcool éthylique.

13. Procédé selon l'une des revendications 1 à 12, caractérisé en ce que les microcapsules sont récupérées à l'état sec par évaporation du ou des solvants de lavage.

14. Procédé selon l'une des revendications 1 à 13, caractérisé en ce que, après leur séparation, les microcapsules sont soumises à une opération de stérilisation, de préférence une radiostérilisation par application d'un rayonnement d'environ 2,5 M rad.

15. Procédé selon l'une des revendications 1 à 14, caractérisé en ce que la substance pharmaceutiquement active adsorbée par les paroi des microcapsules est constituée par un composé hydrosoluble, ayant un poids moléculaire sensiblement compris entre 300 et 600, et portant une charge électrique.

16. Procédé selon la revendication 15, caractérisé en ce que la substance pharmaceutiquement active est la doxorubicine, de préférence sous la forme de son chlorhydrate.

**Patentansprüche**

1. Verfahren zur Herstellung von Mikrokapseln, die eine pharmazeutisch aktive Substanz einschließen, dadurch gekennzeichnet, daß man die Mikrokapseln ausgehend von Serumalbumin durch die nachstehenden aufeinanderfolgenden Arbeitsgänge herstellt:

a) Serumalbumin wird durch Dispersion in einem organischen Lösungsmittelsystem durch Zusatz eines pharmazeutisch brauchbaren Emulgiermittels und Bewegen emulgiert;

b) zu der so erhaltenen, bewegt gehaltenen Emulsion, fügt man ein Vernetzungsmittel, gelöst in dem genannten organischen Lösungsmittelsystem und bewegt weiter, bis der gewünschte Grenzflächen-Vernetzungsgrad erzielt ist;

c) man isoliert die Mikrokapseln nach Verdünnen des Reaktionsgemischs, worauf Waschstufen mit geeigneten Lösungsmitteln und Dekantierstufen folgen, wonach man die Mikrokapseln mehrfach mit Alkohol wäscht und in Alkohol, vorzugsweise absoluten, während mindestens 12 Stunden eintaucht, um das nicht-vernetzte Serumalbumin zu denaturieren, und

d) nach dem Trocknen der Mikrokapseln führt man die pharmazeutisch aktive Substanz durch gesteuertes Eintauchen der Mikrokapseln in eine titrierte Lösung dieser Substanz ein.

2. Verfahren nach Anspruch 1, dadurch gekennzeichnet, daß man die Mikrokapseln durch die nachstehenden aufeinanderfolgenden Arbeitsgänge isoliert:

man trennt die überstehende Flüssigkeit ab;

man führt mindestens eine Wäsche der Mikrokapseln mit dem genannten organischen Lösungsmittelsystem durch;

man fügt zu den Mikrokapseln der Reihe nach 1/3 Glycerin mit einem Gehalt von etwa 25% Polysorbat und anschließend 2/3 Wasser;

man trennt die Mikrokapseln durch Sedimentation vor der Durchführung der genannten Wäsche mit Alkohol und dem Eintauchen in Alkohol ab.

3. Verfahren nach einem der Ansprüche 1 oder 2, dadurch gekennzeichnet, daß das Emulgiermittel Phosphatidylcholin ist.

4. Verfahren nach einem der Ansprüche 1 bis 3, dadurch gekennzeichnet, daß das organische Lösungsmittelsystem aus einem binären Gemisch eines chlorierten Kohlenwasserstoffs mit $C_1$ oder $C_2$ und eines nichtchlorierten Kohlenwasserstoffs mit $C_5$ bis $C_7$ besteht.

5. Verfahren nach Anspruch 4, dadurch gekennzeichnet, daß das organische Lösungsmittelsystem aus einem Gemisch von Chloroform und Cyclohexan besteht.

6. Verfahren nach einem der Ansprüche 1 bis 5, dadurch gekennzeichnet, daß das Vernetzungsmittel ein bifunktionelles Acylierungs-Reagenz, wie ein Dicarbonsäure-halogenid oder Diisocyanat, ist, in dem die beiden funktionellen Gruppen beide von einem aromatischen Ring getragen werden oder durch eine aliphatische Kette getrennt werden, die 2 bis 10 Kohlenstoffatome aufweist, oder eine von einem aromatischen Ring und die andere von einer aliphatischen, an den Kern angelagerten Kette getragen werden.

7. Verfahren nach Anspruch 6, dadurch gekennzeichnet, daß das Vernetzungsmittel ausgewählt wird aus Succinyldichlorid, Sebacoyldichlorid, Terephthaloyldichlorid, Toluoldiisocyanat und Hexamethylendiisocyanat.

8. Verfahren nach einem der Ansprüche 1 bis 7, dadurch gekennzeichnet, daß die Zeit zur Grenzflächen-Vernetzung bei etwa 30 Minuten bis etwa 90 Minuten liegt.

9. Verfahren nach Anspruch 8, dadurch gekennzeichnet, daß die Zeit für die Grenzflächenvernetzung etwa 60 Minuten beträgt.

10. Verfahren nach einem der Ansprüche 1 bis 9, dadurch gekennzeichnet, daß das Gewichtsverhältnis des Serumalbumins zum Vernetzungsmittel so ausgewählt wird, daß:

$$\frac{1}{1,250} < \frac{Serumalbumin}{Vernetzungsmittel} < \frac{1}{0,2}$$

11. Verfahren nach Anspruch 10, dadurch gekennzeichnet, daß das Gewichtsverhältnis von Serumalbumin zum Vernetzungsmittel so ausgewählt wird, daß:

$$\frac{1}{0{,}7} \quad < \quad \frac{Serumalbumin}{Vernetzungsmittel} \quad < \quad \frac{1}{0{,}2}$$

12. Verfahren nach einem der Ansprüche 1 bis 11, dadurch gekennzeichnet, daß der für die Wasch- und Eintauch-Arbeitsgänge der Mikrokapseln verwendete Alkohol aus Ethylalkohol besteht.

13. Verfahren nach einem der Ansprüche 1 bis 12, dadurch gekennzeichnet, daß die Mikrokapseln im trockenen Zustand durch Verdampfen des oder der Waschlösungsmittel gewonnen werden.

14. Verfahren nach einem der Ansprüche 1 bis 13, dadurch gekennzeichnet, daß die Mikrokapseln nach ihrer Abtrennung einem Sterilisationsarbeitsgang, vorzugsweise einer Radiosterilisation durch Anwendung einer Bestrahlung von etwa 2,5 M rad unterzogen werden.

15. Verfahren nach einem der Ansprüche 1 bis 14, dadurch gekennzeichnet, daß die durch die Wandung der Mikrokapseln adsorbierte pharmazeutisch aktive Substanz aus einer wasserlöslichen Verbindung mit einem Molekulargewicht von 300 bis 600 besteht, die eine elektrische Ladung trägt.

16. Verfahren nach Anspruch 15, dadurch gekennzeichnet, daß die pharmazeutisch aktive Substanz Doxorubicin, vorzugsweise in der Form seines Hydrochlorids ist.

**Claims**

1. Process for preparing microcapsules containing a pharmaceutically active substance, wherein the microcapsules are prepared from serum albumin by carrying out the following successive operations:

a) the serum albumin is emulsified by dispersion in an organic solvent system, by adding a pharmaceutically acceptable emulsifying agent, and stirring;

b) to the emulsion thereby obtained, maintained stirred, a crosslinking agent dissolved in the said organic solvent system is added, and stirring is maintained until the desired degree of interfacial crosslinking is obtained;

c) the microcapsules are isolated after dilution of the reaction mixture followed by a succession of stages of washing with suitable solvents and decantation stages, and the microcapsules are then subjected to several washes with alcohol and to immersion in alcohol, preferably absolute, for at least 12 hours in order to denature the non-crosslinked serum albumin, and

d) after the microcapsules are dried, the pharmaceutically active substance is incorporated therein by controlled immersion of the microcapsules in a titrated solution of the said substance.

2. Process as claimed in claim 1, wherein the microcapsules are isolated by carrying out the following successive operations:

the supernatant is separated;

at least one washing of the microcapsules is carried out using the said organic solvent system;

1/3 of glycerine containing approximately 25% of polysorbate and then 2/3 of water are added to the microcapsules in that order;

the microcapsules are separated by sedimentation, before being washed with alcohol and immersed in alcohol.

3. Process as claimed in claim 1 or 2, wherein the emulsifying agent is phosphatidylcholine.

4. Process as claimed in one of claims 1 to 3, wherein the organic solvent system consists of a binary mixture of a chlorinated $C_1$ or $C_2$ hydrocarbon and a non-chlorinated $C_5$ to $C_7$ hydrocarbon.

5. Process as claimed in claim 4, wherein the organic solvent system consists of a mixture of chloroform and cyclohexane.

6. Process as claimed in one of claims 1 to 5, wherein the crosslinking agent is an acylating bifunctional reagent, such as a dicarboxylic acid halide or a diisocyanate, in which the two functional groups are both carried on an aromatic ring, or alternatively are separated by an aliphatic chain of 2 to 10 carbon atoms, or one of which is carried on an aromatic ring and the other on an aliphatic chain attached to the ring.

7. Process as claimed in claim 6, wherein the crosslinking agent is chosen from succinyl dichloride, sebacoyl dichloride, terephtaloyl dichloride, toluene diisocyanate and hexamethylene diisocyanate.

8. Process as claimed in one of claims 1 to 7, wherein the interfacial crosslinking time is between about 30 minutes and about 90 minutes.

9. Process as claimed in claim 8, wherein the interfacial crosslinking time is about 60 minutes.

10. Process as claimed in one of claims 1 to 9, wherein the ratio by weight of the serum albumin to the crosslinking agent is chosen such that:

$$\frac{1}{1{.}250} \quad < \quad \frac{serum\ albumin}{crosslinking\ agent} \quad < \quad \frac{1}{0{.}2}$$

11. Process as claimed in claim 10, wherein the ratio by weight of the serum albumin to the crosslinking agent is chosen such that:

$$\frac{-1}{0.7} < \frac{\text{serum albumin}}{\text{crosslinking agent}} < \frac{1}{0.2}$$

12. Process as claimed in one of claims 1 to 11, wherein the alcohol used for the operations of washing and immersion of the microcapsules consists of ethyl alcohol.

13. Process as claimed in one of claims 1 to 12, wherein the microcapsules are recovered in the dry state by evaporation of the washing solvent or solvents.

14. Process as claimed in one of claims 1 to 13 wherein, after they are separated, the microcapsules are subjected to a sterilization operation, preferably radio-sterilization by application of radiation of approximately 2.5 Mrad.

15. Process as claimed in one of claims 1 to 14, wherein the pharmaceutically active substance adsorbed by the wall of the microcapsules consists of a water-soluble compound having a molecular weight of substantially between 300 and 600 and bearing an electrical charge.

16. Process as claimed in claim 15, wherein the pharmaceutically active substance is doxorubicin, preferably in the form of its hydrochloride.

FIG_1

FIG_2

FIG_3